# EUROPÄISCHE PATENTANMELDUNG

(11) **EP 4 710 964 A1**
(43) Veröffentlichungstag der Anmeldung: **18.03.2026**
(21) Anmeldenummer: 24200575.9
(22) Anmeldetag: 16.09.2024
(51) Int. Cl.: A61M 5/142, A61M 39/28

(54) **MEDIZINISCHES ÜBERLEITUNGSSYSTEM FÜR EINE MEDIZINISCHE PUMPE, KLEMMENMODUL FÜR EINE MEDIZINISCHE PUMPE, MEDIZINISCHE PUMPE UND VERFAHREN ZUR ERKENNUNG EINES MEDIZINISCHEN ÜBERLEITUNGSSYSTEMS**

(71) Anmelder: B. Braun Melsungen AG, 34212 Melsungen (DE)
(72) Erfinder: ERLEN, Christoph, 34132 Kassel (DE); SCHWARZ, Jan, 34212 Melsungen (DE)
(74) Vertreter: Winter, Brandl - Partnerschaft mbB

(57) **Zusammenfassung**

Offenbart ist ein medizinisches Überleitungssystem (14, 18) zur Verwendung mit einer medizinischen Pumpe (1) zumindest aufweisend: einen Schlauch (14), der dafür vorgesehen und ausgestaltet ist, abschnittsweise in eine Schlauchaufnahme (12) oder Pumpenstrecke der Pumpe (1) eingesetzt zu werden, um ein Fluid von einem Reservoir zu einem Patienten überzuleiten, und eine am Schlauch (14) vorgesehene Schlauchklemme (18) mit einer vorbestimmten Farbe und/oder optischen Markierung, wobei die Schlauchklemme (18) dafür vorgesehen und ausgestaltet ist, zumindest abschnittsweise in die Pumpe (1) eingesetzt zu werden und von einer Lichtquelle (34) der Pumpe (1) mit einem Lichtstrahl (34e) in/ mit Hauptwellenl6ngen (42a, 42b, 42c, 42d) bestrahlt zu werden, um das an der Schlauchklemme (18) reflektierte oder reflektierende Licht (34r) von einer Photodetektoranordnung (38) der Pumpe (1) zu empfangen und in Einzelsignale (46a, 46b, 46c, 46d) der Hauptwellenlängen zu trennen. Offenbart sind weiterhin ein medizinisches Klemmenmodul, eine medizinische Pumpe und ein Verfahren zur Erkennung eines medizinischen Überleitungssystems in einer medizinischen Pumpe.

## Beschreibung

### Technisches Gebiet

Die vorliegende Offenbarung betrifft ein medizinisches Überleitungssystem zur Verwendung mit einer medizinischen Pumpe, ein Klemmenmodul für eine medizinische Pumpe, eine medizinische Pumpe, sowie ein Verfahren zur Erkennung eines medizinischen Überleitungssystems basierend auf einer Farbe einer Schlauchklemme des medizinischen Überleitungssystems.

### Hintergrund der Offenbarung

Ein medizinisches Überleitungssystem oder Infusionsset hat zumindest einen Schlauch und eine daran - meist unlösbar - befestigte Schlauchklemme. Die Schlauchklemme kann als Schiebeklemme ausgestaltet sein und wirkt mit dem Schlauch derart zusammen, dass selektiv ein freier Fluss durch den Schlauch verhindert wird. Häufig wird eine solche Schlauchklemme durch die Pumpe selbst im Rahmen eines Einsetzvorgangs des Überleitungssystems in die Pumpe betätigt, etwa mittels einer Frontklappe der Pumpe bei deren Schließbewegung. Alternativ kann die Schlauchklemme manuell, unabhängig von einem Schließmechanismus der Pumpe betätigbar sein.

Die Schlauchklemme weist typischerweise ein sich verjüngendes Langloch auf, durch das der Schlauch verläuft. Der Schlauch ist an einem Ende des Lochs nicht verschlossen und wird zusammengedrückt, wenn er zum anderen Ende des Lochs geschoben wird. In einigen Fällen dient die als Gleitklemme ausgebildete Schlauchklemme funktional als Schlüssel zum Ein- und Ausschalten der Infusionspumpe und kann dabei helfen, den Schlauch des Überleitungssystems im Pumpenkanal zu befestigen. Bei einigen Pumpen dient die Schlauchklemme/ Schiebeklemme auch als Schlüssel zum Öffnen der Pumpentür, um den Infusionsschlauch zu laden oder zu entladen. Die Pumpentür wird entriegelt, indem ein Teil der Schlauchklemme/ Schiebeklemme in einen Schlitz im Pumpenkörper gedrückt wird. Durch Drücken der Schlauchklemme/ Schiebeklemme in den Schlitz wird der Schlauch zum schmalen Ende des konischen Lochs gedrückt, wodurch der Schlauch geschlossen wird. Somit bietet die Schlauchklemme ein Maß an Sicherheit, indem sie einen freien Fluss verhindert, da sie den Schlauch beim Öffnen der Pumpentür zusammendrückt.

Schlauchklemmen dieser Gattung sind oft passend für genau eine bestimmte Pumpe ausgebildet und stellen so sicher, dass nur kompatible Überleitungssysteme mit dieser Pumpe verwendet werden. Vor diesem Hintergrund ist es von Bedeutung, sicherstellen zu können, dass die richtige Schlauchklemme verwendet wird, bzw. erkennen zu können, welche Art an Überleitungssystem eingesetzt ist.

### Stand der Technik

Aus dem Stand der Technik ist die US 9,272,129 B2 bekannt, in welcher ein Infusionsset bereitgestellt wird, bei dem eine Schiebklemme/ Schlauchklemme durch die Verwendung von Farben, Löchern oder dergleichen kodiert werden kann, um die Art des damit verwendeten Infusionsschlauchs zu identifizieren. Die Infusionspumpe kann den Code auf der Schlauchklemme/ Schiebeklemme erkennen und dadurch den Typ des Infusionsschlauchs bestimmen und dadurch nur den Zugriff auf Infusionsprogramme für Medikamente oder Infusionslösungen ermöglichen, die mit dem jeweiligen Infusionsschlauchtyp kompatibel sind.

Zudem ist aus der US 2006/0224128 A1 ein System und ein Verfahren zum automatischen Verabreichen eines Infusats an einen Patienten offenbart. Das System umfasst ein Infusionsset und ein Infusionsgerät. Eine an einer Infusionsset-Komponente angeordnete Signalisierungskomponente identifiziert ein Verabreichungsprotokoll für das Infusionsset. Eine operativ mit der Infusionsvorrichtung verbundene Erfassungsvorrichtung erfasst die Signalisierungskomponente und identifiziert das Verabreichungsprotokoll. Das Infusionsgerät wird dann so konfiguriert, dass es gemäß dem Verabreichungsprotokoll arbeitet.

Die Druckschriften US 5 290 239 A und US 6 635 033 B1 zeigen jeweils ein optisches Kontrollsystem zur Bestimmung einer korrekten Größe und Funktion einer Schlauchklemme, um bei inkorrekter Schlauchklemme einen Infusionsbetrieb zu unterbinden.

Die Druckschrift DE 10 2021 215 067 A1 offenbart eine medizinische Pumpe mit einem opto-elektrischen System, mittels dem eine eingesetzte Schlauchklemme mit unterschiedlichen Wellenlängen bestrahlt wird und das reflektierte Licht zur Bestimmung der Farbe der Schlauchklemme ausgewertet wird. Hierbei wird auch vorgeschlagen, die Schlauchklemme(n) zur verbesserten Farbunterscheidung mit einem Infrarot-Marker zu versehen.

Der Stand der Technik bringt den Nachteil mit sich, dass ein vorrichtungstechnischer Aufwand zur Bestrahlung der Schlauchklamme oder zur Erfassung des von ihr reflektierten Lichtes hoch ist.

### Kurzbeschreibung der Offenbarung

Vor dem Hintergrund liegt der vorliegenden Offenbarung die objektiv technische Aufgabe zugrunde, die Nachteile aus dem Stand der Technik zu beseitigen oder zumindest zu vermindern und ein medizinisches Überleitungssystem, ein Klemmenmodul, eine medizinische Pumpe und ein Verfahren bereitzustellen, durch das, bzw. die ein Erkennen eines medizinischen Überleitungssystems mit geringerem Aufwand, insbesondere vorrichtungstechnischem Aufwand, erfolgt.

Die Aufgabe wird hinsichtlich des medizinischen Überleitungssystems durch die Merkmale des Patentanspruchs 1, hinsichtlich des Klemmenmoduls durch die Merkmale des Patentanspruchs 4, hinsichtlich der Pumpe durch die Merkmale des Patentanspruchs 9 und hinsichtlich des Verfahrens durch die Merkmale des Patentanspruchs 10 gelöst. Vorteilhafte Weiterbildungen sind in den abhängigen Ansprüchen beschrieben.

Die vorliegende Offenbarung betrifft ein medizinisches Überleitungssystem, insbesondere ein Transfusionsset oder ein Infusionsset, zur Verwendung mit einer medizinischen Pumpe, insbesondere einer Transfusionspumpe, Infusionspumpe, Schmerzpumpe oder enteralen Pumpe, die vorzugsweise als Schlauchpumpe ausgebildet ist. Zumindest hat das Überleitungssystem:
- einen Schlauch, der dafür vorgesehen und ausgestaltet ist, abschnittsweise in eine Schlauchaufnahme oder Pumpenstrecke der Pumpe eingesetzt zu werden, um ein Fluid von einem Reservoir zu einem Patienten überzuleiten, und
- eine am Schlauch, vorzugsweise unlösbar, vorgesehene Schlauchklemme, insbesondere Schiebeklemme, mit einer vorbestimmten Farbe und/oder vorbestimmten optischen Markierung. Gemäß der Offenbarung ist die Schlauchklemme dafür vorgesehen und ausgestaltet, zumindest abschnittsweise in die Pumpe eingesetzt, vorzugsweise eingeschoben, zu werden und von einer Lichtquelle der Pumpe mit einem Lichtstrahl, der ein Spektrum mit unterschiedlichen Hauptwellenlängen, bzw. Hauptfrequenzen, aufweist, bestrahlt zu werden, um in Folge das an der Schlauchklemme reflektierte oder reflektierende Licht von einer Detektoranordnung der Pumpe zu empfangen und von dieser in Einzelsignale der Hauptwellenlängen zu trennen.

Auf diese Weise ist ein medizinisches Überleitungssystem geschaffen, bei dem die Schlauchklemme zum einen nicht sequentiell, sondern stattdessen zeitgleich mit unterschiedlichen Hauptwellenlängen bestrahlt wird, und zum anderen die Einzelsignale der Hauptwellenlängen des reflektierten oder reflektierenden Lichtes nicht sequentiell ermittelt werden, sondern zeitgleich durch die eine Detektoranordnung.

Das offenbarungsgemäß vorgesehene und ausgestaltete, medizinische Überleitungssystem kann somit mit geringerem Aufwand, insbesondere geringerem vorrichtungstechnischem Aufwand, erkannt werden.

Gemäß einer Weiterbildung hat die Schlauchklemme die Farbe Magenta, Grün, Grau, Rot, Weiß, Gelb, Pink, Blau oder Orange, bzw. weisen die Einzelsignale Signalstärken auf, welche in Summe Magenta, Grün, Grau, Rot, Weiß, Gelb, Pink, Blau oder Orange entsprechen.

Gemäß einer Weiterbildung ist die optische Markierung eine Infrarot-Markierung. Insbesondere ist die optische Markierung ein Infrarot-Absorber mit vorbestimmter Konzentration in einem Material der Schlauchklemme, sodass eine Absorption der Schlauchklemme im Infrarot, bzw. eine Reflexion, vorbestimmt und/ oder eingestellt ist.

Des Weiteren betrifft die vorliegende Offenbarung ein Klemmenmodul einer oder für eine medizinische Pumpe, insbesondere eine Transfusionspumpe, Infusionspumpe, Schmerzpumpe oder enterale Pumpe, die vorzugsweise als Schlauchpumpe ausgebildet ist. Das Klemmenmodul hat ein elektro-optisches System oder bildet es aus. Das elektro-optische System ist zum Erkennen einer eine vorbestimmte Farbe und/oder optische Markierung aufweisenden Schlauchklemme eines zumindest abschnittsweise in das Klemmenmodul einsetzbaren, medizinischen Überleitungssystems vorgesehen und ausgestaltet. Insbesondere ist das medizinische Überleitungssystem gemäß wenigstens einem Aspekt der vorangegangenen Beschreibung ausgestaltet. Das elektro-optische System hat zumindest folgende System-Komponenten:
- eine Lichtquelle, die vorgesehen und ausgestaltet ist, einen mehrere Hauptwellenlängen aufweisenden Lichtstrahl auf die Schlauchklemme zu senden, das heißt, die Schlauchklemme in mehreren Hauptwellenlängen zeitgleich zu bestrahlen, und
- eine Photodetektoranordnung, die vorgesehen und ausgestaltet ist, an dem medizinischen Überleitungssystem, insbesondere an der Schlauchklemme, reflektiertes oder reflektierendes Licht zu empfangen. Gemäß der Offenbarung ist die Photodetektoranordnung dafür vorgesehen und ausgestaltet, das reflektierte oder reflektierende Licht in Einzelsignale der Hauptwellenlängen zu trennen. Das heißt, dass die Einzelsignale von der Photodetektoranordnung nicht sequentiell, sondern zeitgleich erfasst werden und zeitgleich getrennt werden. In anderen Worten ist die Photodetektoranordnung spektral-sensitiv vorgesehen und ausgestaltet.

Offenbarungsgemäß ist somit ein Klemmenmodul einer oder für eine medizinische Pumpe, geschaffen, durch welches das Erkennen eines medizinischen Überleitungssystems mit geringerem Aufwand, insbesondere vorrichtungstechnischem Aufwand, erfolgt.

Vorzugsweise hat das Klemmenmodul ein optisches Fenster zur Bündelung des von der Lichtquelle gesendeten Lichtstrahls und/ oder zur Bündelung des von der Schlauchklemme reflektierten oder reflektierenden Lichts hin zu der Photodetektoranordnung. Vorzugsweise ist das Fenster aus Kunststoff.

Gemäß einer Weiterbildung hat die Photodetektoranordnung nur einen (einzigen) Photodetektor, der offenbarungsgemäß spektral-sensitiv vorgesehen und ausgebildet ist, sodass sich ein vorrichtungstechnisch besonders kompaktes Klemmenmodul ergibt.

Gemäß einer Weiterbildung hat die Photodetektoranordnung je einen Photodetektor pro Hauptwellenlänge, woraus sich die offenbarungsgemäße Spektral-Sensitivität der Photodetektoranordnung ergibt.

Gemäß einer Weiterbildung hat oder ist die Lichtquelle eine RGB-LED, vorzugsweise eine kombinierte RGB-LED.

Gemäß einer Weiterbildung hat oder ist die Lichtquelle eine Rot-LED, wobei eine der Hauptwellenlängen Rot mit 630nm ± 16nm ist. Alternativ oder ergänzend hat oder ist die Lichtquelle eine Grün-LED, wobei eine der Hauptwellenlängen Grün mit 520nm ± 33nm ist. Alternativ oder ergänzend hat oder ist die Lichtquelle eine Blau-LED, wobei ein der Hauptwellenlängen Blau mit 467nm ± 25nm ist.

Gemäß einer Weiterbildung hat oder ist die Lichtquelle ergänzend eine Infrarot- oder IR-LED, wobei eine der Hauptwellenlängen nahes Infrarot mit 950nm ± 42nm ist.

Vorzugsweise werden zur differenzierten Farberkennung die RGB-Einzelsignale zusammen mit dem IR- Einzelsignale ausgewertet.

Vorzugsweise sind die Lichtquelle und die Photodetektoranordnung bauraumsparend auf einer gemeinsamen Leiterplatine des Klemmenmoduls angeordnet.

Vorzugsweise ist eine Auswerteeinheit und/ oder Prozessoreinheit des Klemmenmoduls oder der Pumpe vorgesehen und die Auswerteeinheit und/ oder Prozessoreinheit ist ausgestaltet, in Abhängigkeit der Einzelsignale die Farbe der Schlauchklemme zu ermitteln und daran das eingesetzte, medizinische Überleitungssystems zu erkennen, insbesondere dessen Art, Typ und/ oder vorbestimmte Verwendung zu erkennen.

Beim Einsetzen des medizinischen Überleitungssystems in die Pumpe kann somit das eingesetzte Überleitungssystem mittels der Photodetektoranordnung und der Auswerteeinheit oder Prozessoreinheit in Abhängigkeit der Einzelsignale, bzw. der ermittelten Farbe, erkannt werden und insbesondere zur Aktivierung von Parametern in einer Steuereinheit der Pumpe genutzt werden. Diese Parameter sind zum Beispiel
- Parameter zur Steuerung einer Druckerkennung,
- Parameter zur Steuerung einer Lufterkennung,
- Parameter zur Definition einer maximalen Verweildauer des medizinischen Überleitungssystems und/ oder
- Parameter zu Air-Stop-Filter (ja/nein).

Die zu aktivierenden Parameter sind vorzugsweise in einem medizinischen Überleitungssystem-Datensatz hinterlegt.

Gemäß der Offenbarung sind die Hauptwellenlängen vorzugsweise:
Rot: Hauptwellenlänge 630 nm ± 16 nm
Grün: Hauptwellenlänge 520 nm ± 33 nm
Blau: Hauptwellenlänge 467 nm ± 25 nm
Infrarot (IR): Hauptwellenlänge 950 nm ± 42 nm

Bezüglich eines konkreten Materials der Schlauchklemme erweisen sich in folgender Lookup-Table dargestellte Bereiche als bevorzugt. In der Lookup-Tabelle sind für die vorbestimmten, zu erkennenden Farben der Schlauchklemme die vorbestimmten Einzelsignale der Hauptwellenlängen als Reflexionswert in Prozent einer Norm oder einer Referenz abgebildet:

| Farbe | Normierte Remission für blau (Hauptwellenlänge 466 nm) | Normierte Remission für grün (Hauptwellenlänge 518 nm) | Normierte Remission für rot (Hauptwellenlänge 629 nm) |
|---|---|---|---|
| Magenta | [0.0 % - 19.1 %] | [1.2 % - 26.6 %] | [49.8 % .- 74.6 %] |
| Grün | [80.1 % - 91.5 %] | [63.5 % - 81.7 %] | [2.4 % - 25.0 %] |
| Grau | [109.9 % ..113.9 %] | [110.0 % ..113.9 %] | [101.8 % - 104.5 %] |
| Rot (mit IR Absorber) | [0.0 % - 33.4 %] | [0.0 % - 34.8 %] | [163.4 % - 193.7 %] |
| Weiß | [226.7 % - 265.8 %] | [231.9 % - 271.7 %] | [217.2 % - 257.7 %] |
| Gelb (mit IR Absorber) | [108.7 % - 121.6 %] | [4.4 % - 62.2 %] | [118.7 % - 133.8 %] |
| Pink | [30.0 % - 51.7 %] | [63.6 % - 79.5 %] | [49.6 % - 66.5 %] |
| Blau | [5.5 % - 33.7 %] | [44.6 % - 62.7 %] | [0.0 % - 17.6 %] |
| Orange | [14.1% - 42.0%] | [0.0 % - 23.2%] | [101.1 % - 108.8 %] |
| Grün (mit IR-Absorber) | [183.6 % - 555.5 %] | [166.3 % - 420.5 %] | [0.0 % - 71.3 %] |
| Weiß (mit IR-Absorber) | [168.3 % - 580.4 %] | [168.3 % - 568.0 %] | [168.3 % - 516.7 %] |

Die vorbestimmten Einzelsignale der Hauptwellenlängen oder Reflexionswerte sind dabei - wie bereits erwähnt - abhängig von dem Material der Schlauchklemme, sodass sie sich bei einem anderen Materialen ändern. In anderen Worten ausgedrückt, werden die eingestrahlten Hauptwellenlängen an der Schlauchklemme in Abhängigkeit des eingesetzten Materials und einer Farb-/ Pigment-Beimischung der Schlauchklemme mit unterschiedlichem Reflexionsgrad reflektiert. Dieser Reflexionsgrad ist (für ein bestimmtes Material) in Form der Look-up Tabelle in der Pumpe hinterlegt, um über einen relativen Vergleich der Einzelsignale (in R, in G, in B und/ oder in IR) zueinander, die Farbe der Schlauchklemme über die Look-up Tabelle zu ermitteln.

Dies bedeutet, dass wenn die Schlauchklemme ein anderes Material aufweist, eine neue Lookup-Table vorgesehen sein muss. Es ist jedoch bevorzugt, wenn die sich daraus ergebenden Remissionsspektren in ihren Eigenschaften unverändert bleiben.

Des Weiteren betrifft die vorliegende Offenbarung eine medizinische Pumpe, insbesondere eine Transfusionspumpe, Infusionspumpe, Schmerzpumpe oder enterale Pumpe, die vorzugsweise als Schlauchpumpe ausgebildet ist, und die ein Klemmenmodul hat, das gemäß wenigstens einem Aspekt der vorangegangenen Beschreibung hat.

Des Weiteren betrifft die vorliegende Offenbarung ein Verfahren/ einen Messalgorithmus zur Erkennung eines medizinischen Überleitungssystems, das insbesondere gemäß wenigstens einem Aspekt der vorangegangenen Beschreibung ausgestaltet ist, basierend zumindest auf einer Farbe und/ oder optischen Markierung der Schlauchklemme und nach einem Einsetzen des medizinischen Überleitungssystems, vorzugsweise nach einem Einschub der Schlauchklemme, in eine medizinische Pumpe, die insbesondere gemäß wenigstens einem Aspekt der vorangegangenen Beschreibung ausgestaltet ist. Das Verfahren/ der Messalgorithmus hat offenbarungsgemäß folgende Schritte:
- vorzugsweise und vorbereitend: Messung einer Temperatur, insbesondere einer Umgebungstemperatur;
- vorzugsweise und vorbereitend: Messung eines Umgebungslichts und Ermittlung einer Umgebungshelligkeit, insbesondere einem Dunkelwert;
- Senden eines Lichtstrahls in/ mit Hauptwellenlängen, insbesondere des Rot und/ oder Grün und/ oder Blau, vorzugsweise ergänzt um eine Hauptwellenlänge des Nah-Infrarot, auf die Schlauchklemme, mittels einer Lichtquelle der Pumpe, das heißt, die Schlauchklemme wird mittels dem Lichtstrahl zeitgleich in/ mit mehreren Hauptwellenlängen bestrahlt oder beleuchtet,
- Empfangen des an der Schlauchklemme reflektierten oder reflektierenden Lichts, über eine Photodetektoranordnung der Pumpe,
- Trennen des reflektierten oder reflektierenden Lichts in Einzelsignale der Hauptwellenlängen, über die Photodetektoranordnung, das heißt, die Photodetektoranordnung ist spektral-sensitiv und über sie erfolgt ein zeitgleiches Trennen der Einzelsignale der Hauptwellenlängen,
- Bestimmen der Farbe der Schlauchklemme durch einen Abgleich der Einzelsignale mit einer in der medizinischen Pumpe hinterlegten Lookup-Tabelle/ Auswertetabelle, und
- Erkennen des Überleitungssystems anhand der Farbe.

Es ist bevorzugt, wenn bereits Kalibrierdaten in einer Auswerteeinheit und/ oder Prozessoreinheit des Klemmenmoduls oder der Pumpe vorgesehen sind, welche zur Initialisierung des Verfahrens/ des Messalgorithmus verwendet werden, und die Lookup-Tabelle/ Auswertetabelle eine optische Charakterisierung der eingesetzten Schlauchklemme bezüglich derer Reflexion aufweist und mittels eines darin hinterlegten Wertebereichs Schwankungen der Messung ausgleichbar sind.

In anderen Worten ist es vorgesehen, dass in eine Berechnung der Einzelsignale ein von der Detektoranordnung erfasstes Remissionsspektrum der Schlauchklemme eingeht. Die Einzelsignale der Hauptwellenlängen liegen als sogenannte "Counts" vor und sind insbesondere Analog-zu-Digital-Messwerte der Photodetektoranordnung. Die Einzelsignale sind weiterhin abhängig von den Kalibrierdaten (insbesondere einem Weißabgleich), der Umgebungstemperatur und dem Dunkelwert (Umgebungslichteinfluss). Daraus resultiert eine nachfolgende Berechnungsgrundlage.

Eine Eingangsvoraussetzung zur Durchführung des Verfahrens/ des Messalgorithmus ist das Vorliegen der Kalibrierdaten, vorzugsweise inklusive der Temperaturmessung. Die Kalibrierdaten werden zur "Initialisierung" des Verfahrens/ des Messalgorithmus benötigt. Die Kalibrierdaten erzeugen einen Reflektionskoeffizienten über ein Weißnormal, der in die Berechnung einfließt und in der Gesamtnäherung abhängig von Temperatur und Lichtstärke ist.

Ferner werden nachfolgende Messwerte aufgezeichnet. Ein erster Messwert ist die Temperaturmessung und ein zweiter Messwert ist die Beleuchtung der Schlauchklemme in den Hauptwellenlängen (RGB). Der zweite Messwert betrifft Analog/Digital-Werte pro Farbe. In einem weiteren Messwert wird der Dunkelwert aufgezeichnet, welcher einen Messwert beschreibt, der an der Photodetektoranordnung ohne eine Ausleuchtung durch die Lichtquelle anliegt. Es ist ebenfalls nötig, Licht, das von außen in das Klemmenmodul eindringen kann, bei der Messung zu kompensieren (entspricht dem vorstehenden Umgebungslicht). Daraufhin erfolgt eine Korrektur des Einzelsignals bezüglich des Dunkelwerts. Die Einzelsignale selbst sind diejenigen der Photodetektoranordnung zu einem Messzeitpunkt für jede einzelne Farbe.

Anschließend folgt eine Temperaturkompensation der Einzelsignale über ein Polynom, das die Temperaturverläufe der Lichtquelle, insbesondere der LEDs, annähert. Das heißt, die Lichtquelle und/ oder ihr Lichtstrom verhalten sich für unterschiedliche Temperaturen unterschiedlich. Daher wird der Verlauf dieser Temperaturdegradation für jede Farbe über eine polynomisch gefittete Temperaturkurve der LED-Farben (Polynom 3. Grades) angenähert. Basierend darauf wird pro Farbe eine Reflexions- oder Reflektivitätsberechnung durchgeführt, welche auf die Kalibrierdaten normiert ist. Diese Reflexions- oder Reflektivitätswerte werden pro Hauptwellenlänge mit den in der Lookup-Tabelle oder Auswertetabelle hinterlegten Minimum- und Maximum-Werten verglichen. Der Vergleich berücksichtigt zusätzliche, mögliche Verschmutzungen, weshalb bei der Farbzuordnung das relative Verhältnis aus Mess- und Auswertetabelle-Wert betrachtet wird. Hierbei können entweder alle Messwerte der vier Hauptwellenlängen (RGB und IR) in die Farbbestimmung eingehen. Alternativ ist auch denkbar, die Intensität/ Amplitude des Skalierungsfaktors zu wählen, wenn es keine eindeutige Farbzuordnung geben sollte.

Die Lookup-Table wird mit der Materialqualifizierung der Schlauchklemme erzeugt und vermessen. Sie beinhaltet die optische Charakterisierung des eingesetzten Materials bezüglich ihrer Reflexion der Farben rot, grün, blau und IR. Die Auswertetabelle umfasst einen Wertebereich (min./max. Wert), um Schwankungen bei der Farbemessung ausgleichen zu können. Weitere Informationen zu den Wellenlängen sind vorstehend bereits beschrieben worden. Das Einbringen eines IR-Anteils ist nötig, um beispielsweise die Farben Rot und Gelb von anderen Farben mit identischen Reflexionseigenschaften (Remissionsspektrum) unterscheiden zu können. Ohne die eingebrachten IR-Marker, welche homogen in die Schlauchklemme/ Schiebeklemme eingebracht sind, wären die Remissionsspektren zwar in ihrer Amplitude unterschiedlich, jedoch der eigentliche Verlauf und das Verhältnis aller Farben zueinander identisch. Eine eindeutige Erkennung wäre dadurch nicht gegeben.

Zusammenfassend beruht das Verfahren, bzw. der Messalgorithmus auf der Idee, die Schlauchklemme des medizinischen Überleitungssystems innerhalb der Pumpe mit nur einer Lichtquelle zu beleuchten, die ein Spektrum in wenigstens zwei Hauptwellenlängen hat. Der so emittierte Lichtstrahl wird an der Schlauchklemme reflektiert und das reflektierte Licht wird von der spektral-sensitiven Photodetektoranordnung in Einzelsignale der Hauptwellenlängen getrennt. In Abhängigkeit der Einzelsignale kann die Farbe oder können unterschiedliche Farben der Schlauchklemme ermittelt werden.

Es ist mindestens einmalig nötig, eine Kalibrierung des elektro-optischen Systems durchzuführen, um die Einflüsse einer Einbausituation, beispielsweise einer Toleranz der Einbaulagen der Lichtquelle und der Photodetektoranordnung, sowie elektronische Toleranzen der System-Komponenten auszugleichen. Durch die Kalibrierung wird ein Offset-Wertepaar generiert, das im Verfahren oder Messalgorithmus zur Kompensation verwendet wird.

### Kurzbeschreibung der Figuren

Fig. 1 ist eine Darstellung einer medizinischen Schlauchpumpe mit eingesetztem, medizinische Überleitungssystem gemäß einer Ausführungsform der vorliegenden Offenbarung, in perspektivischer Ansicht von vorne;
Fig. 2 ist eine Darstellung der medizinischen Schlauchpumpe gemäß Figur 1 mit geöffneter Frontklappe und mit eingesetztem, medizinischem Überleitungssystem, in perspektivischer Ansicht von vorne;
Fig. 3 ist eine Darstellung des medizinischen Überleitungssystems gemäß Figur 1 und 2 mit einem Schlauch und einer als Schiebeklemme ausgestalteten Schlauchklemme, die eine vorbestimmte Farbe aufweist;
Fig. 4 ist eine Darstellung eines freigestellten Klemmenmoduls der medizinischen Schlauchpumpe gemäß Figur 1 und 2;
Fig. 5 ist eine Darstellung eines Höhenschnitts des Klemmenmoduls gemäß Figur 4, in perspektivischer Darstellung;
Fig. 6 ist eine Darstellung einer freigestellten Leiterplatine des Klemmenmoduls gemäß Figur 4 und 5, in einer Seitenansicht;
Fig. 7 ist eine Darstellung eines Längsschnitts des Klemmenmoduls gemäß den Figuren 4 und 5, mit Blick auf ein elektro-optisches Systems des Klemmenmoduls;
Fig. 8 ist eine Darstellung des Klemmenmoduls gemäß den Figuren 4, 5 und 7 in einer Seitenansicht, mit einem rückwärtigen Blick auf die Leiterplatine;
Fig. 9 ist eine schematische Darstellung eines Verfahrens/ eines Messalgorithmus zur Erkennung des in die medizinische Schlauchpumpe eingesetzten, medizinischen Überleitungssystems, anhand einer Farbe der Schlauchklemme, gemäß der vorliegenden Offenbarung.

### Beschreibung der Ausführungsbeispiele

Nachstehend werden Ausführungsbeispiele der vorliegenden Offenbarung auf der Basis der zugehörigen Figuren beschrieben.

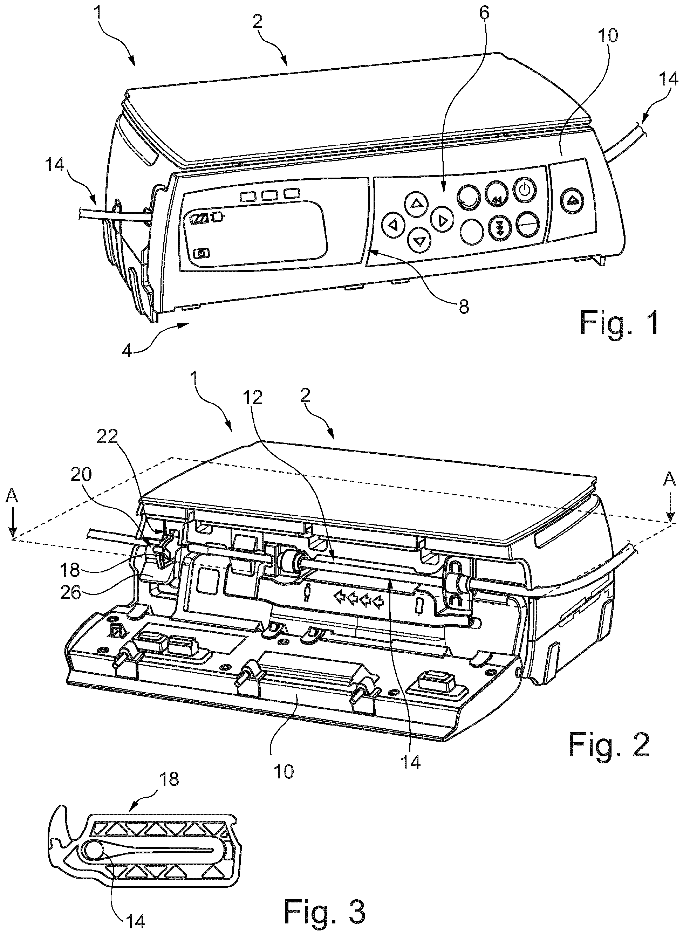

Figur 1 zeigt eine als Infusionsgerät oder Infusionspumpe ausgestaltete medizinische Schlauchpumpe 1 von vorne. Die Schlauchpumpe 1 hat ein Gehäuse 2 mit einer Frontseite 4 und einem unterseitig anscharnierten Schlauchfachdeckel 10, an dem ein Bedienfeld 6 und ein Display 8 angeordnet sind. Vom Schlauchfachdeckel 10 ist ein Schlauchfach 12 - auch als Schlauchaufnahme oder Pumpenstrecke bezeichnet - verschlossen, in das ein Infusionsschlauch 14 eines offenbarungsgemäßen, medizinischen Überleitungssystems 14, 18 eingelegt ist (vgl. Figur 2).

Figur 2 zeigt die medizinische Schlauchpumpe 1 gemäß Figur 1 mit aufgeklapptem Schlauchfachdeckel 10. Gemäß Figur 2 erstreckt sich von rechts, ausgehend von einem Infusionsbehälter (nicht dargestellt) des Überleitungssystems 14, 18, der Infusionsschlauch 14 in das längliche Schlauchfach 12 hinein. Durch Schließen des Schlauchfachdeckels 10 wird der Infusionsschlauch 14 im Schlauchfach 12 festgelegt. Der im Betrieb geschlossene Schlauchfachdeckel 10 (vgl. Zustand Fig. 1) fixiert den Infusionsschlauch 14 in seiner Betriebslage und bildet eine Wandung, gegen die rückwärtig des Schlauchfachs 12 angeordnete Verdränger (nicht dargestellt) den Infusionsschlauch 14 in peristaltischer Abfolge quetschen, um so das zu infundierende Fluid hin zu dem Patienten zu fördern. Das Schlauchfach 12 wird deshalb auch manchmal als Pumpenstrecke bezeichnet und ist so Teil der Peristaltik der Schlauchpumpe 1.

Um sicherzustellen, dass der Fluidfluss zum Patienten ausschließlich durch den Betrieb der Schlauchpumpe 1 und nicht vorzeitig - beispielsweise durch Schwerkraftwirkung bereits beim Einlegen des Infusionsschlauches 14 in das Schlauchfach 12 - erfolgt, ist ein redundantes Klemmensystem vorgesehen.

Zum einen ist hierzu an dem Infusionsschlauch 14 selbst eine offenbarungsgemäße Schlauchklemme 18 unlösbar vorgesehen. Zum anderen verfügt die Schlauchpumpe 1 über ein Klemmenmodul 20 mit einem maschineneigenen Klemmenmechanismus (nicht dargestellt). Der Klemmenmechanismus wird beim Einlegen des Infusionsschlauches 14 durch das Einsetzen und Einschieben der Schlauchklemme 18 in eine Aufnahme 22 des Klemmenmoduls 20 und das darauffolgende Schließen des Schlauchfachdeckels 10 aktiviert.

Fig. 3 zeigt das medizinische Überleitungssystem gemäß Figur 1 und 2 mit dem Schlauch 14 und der als Schiebeklemme ausgestalteten Schlauchklemme 18, die eine vorbestimmte Farbe aufweist. Ergänzend zur vorbestimmten Farbe weist die offenbarungsgemäße Schlauchklemme 18 eine optische Markierung auf, die im gezeigten Ausführungsbeispiel eine Beimischung eines im Nah-Infrarot wirksamen Absorbers, in vorbestimmter Konzentration, zu dem Material der Schlauchklemme 18 ist. Diese optische Markierung stellt, neben der vorbestimmten Farbe, ein ergänzendes Differenzierungsmerkmal der Schlauchklemme 18 dar, anhand dessen die Schlauchklemme 18 - und in Folge das medizinische Überleitungssystem 14, 18 - offenbarungsgemäß eindeutig identifizierbar/ erkennbar ist/ sind.

Die vorbestimmte Farbe der Schlauchklemme 18 wird offenbarungsgemäß beim/ nach dem Einsetzen/ Einschieben der Schlauchklemme 18 in die Aufnahme 22 des Klemmenmoduls 20 der Schlauchpumpe 1 (vgl. Fig. 2) mittels einem elektro-optischen System des Klemmenmoduls 20 erfasst/ ermittelt/ erkannt. In Abhängigkeit der erfassten/ ermittelten/ erkannten Farbe kann in Folge ein der Farbe zugeordneter Betriebsparameter der Schlauchpumpe 1 aktiviert/ freigegeben/ initialisiert werden.

Zur Aufnahme und zum Klemmen des Schlauchs 14 hat die Schlauchklemme 18 ein sich verjüngendes Langloch, in dessen erweiterten Bereich der Schlauch 14 liegt, wenn die Schlauchklemme 18 noch nicht in die Aufnahme 22 eingeschoben ist. Wird die Schlauchklemme 18 in die Aufnahme 22 eingeschoben/ eingesetzt eingelegt, betätigt sie den maschineneigenen Klemmmechanismus, durch den der Infusionsschlauch 14 von der Schlauchklemme 18 abgeklemmt wird, sodass ein unkontrollierter Fluid-Fluss vor einem Start der Schlauchpumpe 1 unterbunden ist. Durch das Öffnen des Schlauchfachdeckels 10 wird die Schlauchklemme 18 hingegen an ihrem - in Figur 3 links angeordneten - Haken über den Infusionsschlauch 14 gezogen (in Fig. 3 von rechts nach links) und verschließt diesen damit.

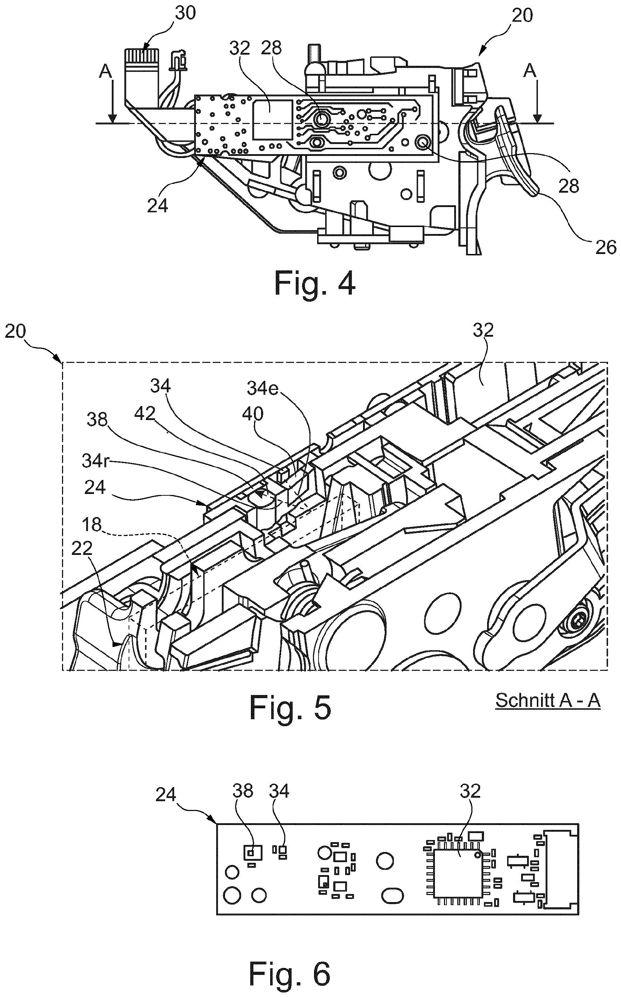

Fig. 4 ist eine Darstellung des freigestellten Klemmenmoduls 20 der medizinischen Schlauchpumpe 1 gemäß Figur 1 und 2. Das Klemmenmodul 20 hat eine Leiterplatte/ - platine 24, die mit Schrauben 28 an einem Rahmen/ einer Tragstruktur des Klemmenmoduls 20 befestigt ist. Auf der Leiterplatine 24 sind Komponenten eines elektro-optischen Systems des Klemmenmoduls 20 platziert, unter anderem ein weiter unten noch näher erläutertes Auswertemodul 32. Die Leiterplatine 24 hat ein Flachbandkabel 30, über das sie mit einem Mainboard/ mit einer Hauptplatine der Schlauchpumpe verbunden ist/ verbunden werden kann.

Fig. 5 ist eine Darstellung des in Figur 2 definierten Höhenschnitts A-A des Klemmenmoduls 20 gemäß den Figuren 1, 2 und 4. Das Klemmenmodul 20 ist hierin freigestellt. Der Schnitt A-A verläuft unterhalb des Schlauchs 14 gemäß Figur 2, sodass dieser in Figur 5 nicht zu sehen ist. Die perspektivische Darstellungsrichtung ist etwa die gleiche wie in Figur 2. Demgemäß erstreckt sich die Aufnahme 22 des Klemmenmoduls 20 in Figur 5 von links unten nach rechts oben. In die Aufnahme 22 ist die Schlauchklemme 18 eingeschoben (gestrichelte Darstellung).

An dem Rahmen/ der Tragstruktur des Klemmenmoduls 20 ist in Figur 5 rückwärtig die Leiterplatte 24 befestigt. Eine Lichtquelle des elektro-optischen Systems des Klemmenmoduls 20 ist als kombinierte RGB-IR-LED 34 ausgebildet und emittiert einen Lichtstrahl 34e auf die eingeschobene Schlauchklemme 18 in Hauptwellenlängen Rot, Grün und Blau des sichtbaren Spektrums, sowie in einer Hauptwellenlänge des nahen Infrarot. Das heißt, die verschiedenen Hauptwellenlängen werden zeitgleich - anstatt sequentiell - emittiert.

Eine Photodetektoranordnung 38 des elektro-optischen Systems des Klemmenmoduls 20 ist auf der Leiterplatine 24 eng benachbart zur Lichtquelle 34 angeordnet und empfängt das von der Schlauchklemme 18 reflektierte/ reflektierende Licht 34r. Das von der Schlauchklemme 18 reflektierte/ reflektierende Licht 34r trifft auf die Photodetektoranordnung 38, die mit dem Auswertemodul 32 des elektro-optischen Systems des Klemmenmoduls 20 signalverbunden ist.

Die Photodetektoranordnung ist im gezeigten Ausführungsbeispiel von (nur) einem spektral-sensitiven Photodetektor 38 gebildet. Das heißt, dass der Photodetektor 38 vorgesehen und ausgestaltet ist, das in den Hauptwellenlängen des Rot, des Grün, des Blau und des nahen Infrarot zeitgleich auf ihn treffende Licht 34r in Einzelsignale der Hauptwellenlängen des Rot, des Grün, des Blau und des nahen Infrarot zu trennen. Alternativ kann die Photodetektoranordnung mehrere Photodetektoren aufweisen, insbesondere einen pro Hauptwellenlänge.

Diese Einzelsignale werden in Folge an das signalverbundene Auswertemodul 32 des elektro-optischen Systems des Klemmenmoduls 20 übertragen. Im Auswertemodul 32 ist eine Lookup-Tabelle abgelegt, in welcher für vorbestimmte Farben der Schlauchklemme 18 vorbestimmte Intervalle der Einzelsignale in den Hauptwellenlängen als Reflexionswert gelistet sind. Unter Abgleich der Einzelsignale mit den in der Lookup-Tabelle gelisteten Intervalle ermittelt so das Auswertemodul 32 die Farbe der Schlauchklemme 18 und ermittelt/ erkennt anhand der Farbe das eingelegte, medizinische Überleitungssystem 14, 18.

Gemäß Figur 5 ist zwischen der Schlauchklemme 18 einerseits und der Lichtquelle 34 und Photodetektoranordnung 38 andererseits ein zweiteiliges optisches Fenster 40, 42 vorgesehen. Ein Abschnitt 40 des optischen Fensters 40, 42 ist ausgebildet, den Lichtstrahl 34e bestimmungsgemäß auf die Schlauchklemme 18 zu richten. Der andere Abschnitt 42 des optischen Fensters 40, 42 ist als Kunststofflinse ausgebildet, über die das von der Schlauchklemme 18 diffus reflektierte/ reflektierende Licht 34r gebündelt auf die Photodetektoranordnung 38 gerichtet wird.

Fig. 6 ist eine Darstellung der freigestellten Leiterplatte 24 des Klemmenmoduls 20 mit dem Auswertemodul 32, der Lichtquelle 34 und der Photodetektoranordnung 38 des elektro-optischen Systems des Klemmenmoduls 20.

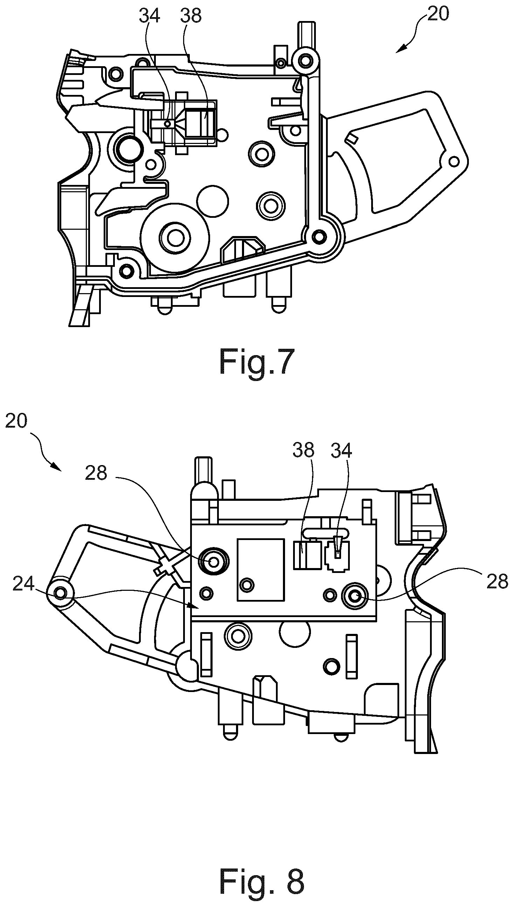

Fig. 7 ist eine Darstellung eines Längsschnitts des Klemmenmoduls 20 mit Blick auf die Lichtquelle 34 und die Photodetektoranordnung 38 des elektro-optischen Systems des Klemmenmoduls 20.

Fig. 8 ist eine Darstellung des Klemmenmoduls 20 mit einem rückwärtigen Blick auf die Leiterplatine 24 und die Lichtquelle 34 und die Photodetektoranordnung 38 des elektro-optischen Systems des Klemmenmoduls 20.

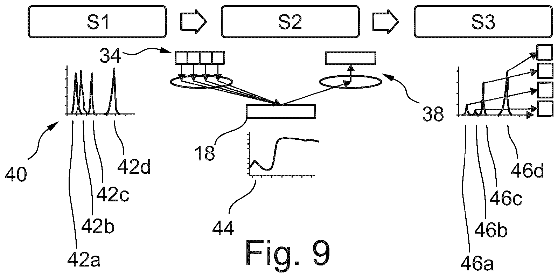

Fig. 9 ist eine Darstellung eines Blockschaltbildes eines offenbarungsgemäßen Verfahrens/ eines offenbarungsgemäßen Messalgorithmus zur Erkennung des oben beschriebenen, medizinischen Überleitungssystems 14, 18, basierend auf der Farbe der Schlauchklemme 18 und nach dem Einsetzen des medizinischen Überleitungssystems 14, 18 und Einschub der Schlauchklemme 18 in die Aufnahme 22 des Klemmenmoduls 20 der medizinischen Pumpe 1. Vorzugsweise erfolgt zunächst die Messung einer Temperatur/ Umgebungstemperatur, um so eine Temperaturabhängigkeit der Lichtquelle 34 und der Photodetektoranordnung 38 temperatur-kompensieren zu können. Ebenso erfolgt vorzugsweise zunächst die Messung des Umgebungslichts und die Ermittlung einer Umgebungshelligkeit, um so die Randbedingungen der Beleuchtung kompensieren zu können.

Offenbarungsgemäß erfolgt gemäß dem Verfahren/ Messalgorithmus gemäß Figur 9 ein Schritt **S1** Senden des Lichtstrahls 34e in/ mit den Hauptwellenlängen 42a, 42b, 42c und 42d - bzw. in/ mit den entsprechenden Hauptfrequenzen - auf die Schlauchklemme 18, mittels der als kombinierte RGB-IR-LED ausgebildeten Lichtquelle 34 der Pumpe 1.

Offenbarungsgemäß wird somit die Schlauchklemme 18 zeitgleich (anstatt sequentiell) in/ mit den Hauptwellenlängen 42a, 42b, 42c und 42d des nahen Infrarot IR, des Blau B, des Grün G und des Rot R bestrahlt. Die Hauptwellenlängen 42a, 42b, 42c und 42d stellen gemäß Figur 9 das Beleuchtungsspektrum 40 des Lichtstrahles 34e dar.

In einem weiteren Schritt **S2** erfolgen ein Reflektieren **S2.1** des Lichtes 34r an der Schlauchklemme 18 mit einem Remissionsspektrum 44 und ein Empfangen **S2.2** des an der Schlauchklemme 18 reflektierten Lichts 34r über die Photodetektoranordnung 38.

In einem weiteren Schritt **S3** erfolgen ein Trennen **S3.1** des reflektierten Lichts 34r in Einzelsignale 46a, 46b, 46c, 46d der Hauptwellenlängen, über die Photodetektoranordnung 38, ein Bestimmen **S3.2** der Farbe durch einen Abgleich der Einzelsignale 46a, 46b, 46c, 46d mit der in der medizinischen Pumpe 1 hinterlegten Auswertetabelle oder Lookup-Tabelle, und das Erkennen **S3.3** des Überleitungssystems 14, 18 anhand der Farbe.

Es ist bevorzugt, wenn als Material der Schlauchklemme ein thermoplastischer Kunststoff auf Basis von Polyolefin oder Polyoxymethylen verwendet werden.

### Bezugszeichen

- 1: Medizinische Pumpe
- 2: Gehäuse
- 4: Frontseite
- 6: Bedienfeld
- 8: Display
- 10: Schlauchfachdeckel
- 12: Schlauchfach
- 14: Schlauch (Überleitungssystem)
- 18: Schlauchklemme (Überleitungssystem)
- 20: medizinisches Klemmenmodul
- 22: Aufnahme
- 24: Leiterplatte
- 26: Tasthebel
- 28: Schraube
- 32: Auswertemodul
- 34: Lichtquelle
- 34e: Lichtstrahl
- 34r: reflektiertes Licht
- 38: Photodetektoranordnung
- 40: Beleuchtungsspektrum
- 42a - d: Emissionssignal Hauptwellenlänge
- 44: Remissionsspektrum Schlauchklemme
- 46a - d: Einzelsignal Hauptwellenlänge
- S1: Schritt Senden Lichtstrahl
- S2.1: Schritt Reflektieren Licht
- S2.2: Schritt Empfangen Licht
- S3.1: Schritt Trennen Einzelsignale
- S3.2: Schritt Bestimmen Farbe
- S3.3: Schritt Erkennen Überleitungssystem

## Patentansprüche

1. Medizinisches Überleitungssystem (14, 18) zur Verwendung mit einer medizinischen Pumpe (1) zumindest aufweisend:
- einen Schlauch (14), der dafür vorgesehen und ausgestaltet ist, abschnittsweise in eine Schlauchaufnahme (12) oder Pumpenstrecke der Pumpe (1) eingesetzt zu werden, um ein Fluid von einem Reservoir zu einem Patienten überzuleiten, und
- eine am Schlauch (14) vorgesehene Schlauchklemme (18) mit einer vorbestimmten Farbe und/oder optischen Markierung, wobei die Schlauchklemme (18) dafür vorgesehen und ausgestaltet ist, zumindest abschnittsweise in die Pumpe (1) eingesetzt zu werden und von einer Lichtquelle (34) der Pumpe (1) mit einem Lichtstrahl (34e) in/ mit Hauptwellenlängen (42a, 42b, 42c, 42d) bestrahlt zu werden, um das an der Schlauchklemme (18) reflektierte oder reflektierende Licht (34r) von einer Photodetektoranordnung (38) der Pumpe (1) zu empfangen und in Einzelsignale (46a, 46b, 46c, 46d) der Hauptwellenlängen zu trennen.

2. Medizinisches Überleitungssystem (14, 18) nach Anspruch 1, **dadurch gekennzeichnet, dass** die Farbe Magenta, Grün, Grau, Rot, Weiß, Gelb, Pink, Blau oder Orange ist.

3. Medizinisches Überleitungssystem (14, 18) nach Anspruch 1 oder 2, **dadurch gekennzeichnet, dass** die optische Markierung von einem Infrarot-Absorber gebildet ist.

4. Medizinisches Klemmenmodul (20) einer oder für eine medizinische Pumpe (1), welches ein elektro-optisches System ausbildet oder hat, das zum Erkennen einer eine vorbestimmte Farbe und/oder optische Markierung aufweisenden Schlauchklemme (18) eines zumindest abschnittsweise in das Klemmenmodul (20) einsetzbaren, medizinischen Überleitungssystems (14, 18) vorgesehen und ausgestaltet ist, zumindest mit folgenden System-Komponenten:
- einer Lichtquelle (34), die vorgesehen und ausgestaltet ist, einen Lichtstrahl (34e) in/ mit, insbesondere wenigstens zwei, Hauptwellenlängen (42a, 42b, 42c, 42d) auf die Schlauchklemme (18) zu senden, und
- einer Photodetektoranordnung (38), die vorgesehen und ausgestaltet ist, an dem medizinischen Überleitungssystem (18), insbesondere an der Schlauchklemme (18), reflektiertes oder reflektierendes Licht (34r) zu empfangen, **dadurch gekennzeichnet, dass** die Photodetektoranordnung (38) vorgesehen und ausgestaltet ist, das reflektierte oder reflektierende Licht (34r) in Einzelsignale (46a, 46b, 46c, 46d) der Hauptwellenlängen zu trennen.

5. Medizinisches Klemmenmodul (20) nach Anspruch 4, **dadurch gekennzeichnet, dass** die Photodetektoranordnung (38) einen einzigen, insbesondere spektral-sensitiven, Photodetektor hat, oder dass die Photodetektoranordnung je einen, insbesondere spektral-spezifischen, Photodetektor pro Hauptwellenlänge hat.

6. Medizinisches Klemmenmodul (20) nach Anspruch 4 oder 5, **dadurch gekennzeichnet, dass** die Lichtquelle (34) eine RGB-LED, vorzugsweise eine kombinierte RGB-LED, ist oder hat.

7. Medizinisches Klemmenmodul (20) nach einem der Ansprüche 4 bis 6, **dadurch gekennzeichnet, dass** die Lichtquelle (34) eine Rot-LED ist oder hat, um den Lichtstrahl (34e) in/ mit einer roten Hauptwellenlänge von 630nm ± 16nm zu senden, und/ oder eine Grün-LED ist oder hat, um den Lichtstrahl (34) in/ mit einer grünen Hauptwellenlänge von 520nm ± 33nm zu senden, und/ oder eine Blau-LED ist oder hat, um den Lichtstrahl (34e) in/ mit einer blauen Hauptwellenlänge von 467nm ± 25nm zu senden.

8. Medizinisches Klemmenmodul (20) nach einem der Ansprüche 4 bis 7, **dadurch gekennzeichnet, dass** die Lichtquelle (34) ergänzend eine IR-LED ist oder hat, um den Lichtstrahl (34e) in/ mit einer Wellenlänge von 950nm ± 42nm, vorzugsweise im Nahen Infrarot, zu senden.

9. Medizinische Pumpe (1), insbesondere Transfusionspumpe, Infusionspumpe, Schmerzpumpe oder enterale Pumpe, insbesondere Schlauchpumpe, mit einem Klemmenmodul (20) gemäß einem der Ansprüche 4 bis 8.

10. Verfahren zur Erkennung eines medizinischen Überleitungssystems (14, 18), das insbesondere gemäß einem der Ansprüche 1 bis 3 ausgestaltet ist, basierend auf einer Farbe einer Schlauchklemme (18) des Überleitungssystems (14, 18), nach einem Einsetzen des medizinischen Überleitungssystems (14, 18), vorzugsweise nach einem Einschub der Schlauchklemme (18), in eine medizinische Pumpe (1), die insbesondere nach Anspruch 9 ausgestaltet ist, **gekennzeichnet durch** Schritte:
- Senden (S1) eines Lichtstrahls (34e) in/ mit Hauptwellenlängen (42a, 42b, 42c, 42d) auf die Schlauchklemme (18), mittels einer Lichtquelle (34) der Pumpe (1), insbesondere zeitgleiches Senden der Hauptwellenlängen,
- Reflektieren (S2.1) von Licht (34r) an der Schlauchklemme (18) mit einem Remissionsspektrum (44),
- Empfangen (S2.2) des an der Schlauchklemme (18) reflektierten Lichts (34r), über eine Photodetektoranordnung (38) der Pumpe (1), insbesondere zeitgleiches Empfangen der Hauptwellenlängen,
- Trennen (S3.1) des reflektierten Lichts (34r) in Einzelsignale (46a, 46b, 46c, 46d) der Hauptwellenlängen, über die Photodetektoranordnung (38), insbesondere zeitgleiches Trennen in die Einzelsignale,
- Bestimmen (S3.2) der Farbe durch einen Abgleich der Einzelsignale (46a, 46b, 46c, 46d) mit einer in der medizinischen Pumpe (18) hinterlegten Auswertetabelle oder Lookup-Tabelle, und
- Erkennen (S3.3) des Überleitungssystems (14, 18) anhand der Farbe.
